## Europäisches Patentamt

## European Patent Office

## Office européen des brevets

(11) Publication number: **0 115 384**
**B1**

# (12) EUROPEAN PATENT SPECIFICATION

(45) Date of publication of patent specification: **08.07.87**

(21) Application number: **84300101.7**

(22) Date of filing: **09.01.84**

(51) Int. Cl.⁴: **A 61 F 2/12**

(54) Mammary prosthesis having adjustable projection

(30) Priority: **20.01.83 US 459383**

(43) Date of publication of application:
**08.08.84 Bulletin 84/32**

(45) Publication of the grant of the patent:
**08.07.87 Bulletin 87/28**

(84) Designated Contracting States:
**DE FR GB IT**

(56) References cited:
EP-A-0 054 197
FR-A- 955 193
FR-A-2 199 266
GB-A-2 073 289
US-A-2 851 692
US-A-3 559 214
US-A-3 911 503

(73) Proprietor: **Medical Engineering Corporation**
**3037 Mt. Pleasant Street**
**Racine Wisconsin 53404 (US)**

(72) Inventor: **LaForest, Lance J.**
**7807 West Coventry**
**Franklin Wisconsin 53122 (US)**
Inventor: **Taylor, Rita L.**
**2206 North Main Street**
**Racine Wisconsin 53402 (US)**

(74) Representative: **Wynne-Jones, John Vaughan
et al**
**Wynne-Jones, Lainé & James 22, Rodney Road
Cheltenham Gloucestershire GL50 1JJ (GB)**

Courier Press, Leamington Spa, England.

## Description

This invention relates generally to the field of medical implants and, more particularly, to mammary prostheses.

Augmentation mammoplasty, that is, surgical augmentation of the breasts, is a common cosmetic surgical procedure that has been performed for many years. This procedure usually entails making a surgical incision to create a pocket in the breast and then inserting a mammary prosthesis, generally shaped similar to the human breast, into the pocket.

Mammary prostheses are well known in the art and generally take several forms. There are single unitary mammary prostheses which comprise a shell of physiologically inert material, such as silicon rubber or the like which are filled with a silicone gel or a saline solution and then sealed. Inflatable mammary prostheses are also available and generally include a hollow shell of physiologically inert material such as silicone rubber which is filled with a saline solution during surgery to achieve the appropriate prosthesis and breast size. In addition to the single shell inflatable mammary prosthesis, an inflatable bilumen or double shell mammary prosthesis is also available. The inflatable bilumen mammary prosthesis generally includes an inner gel implant filled within silicone gel and then sealed. The inner gel implant is disposed within a partially fluid filled, inflatable shell that is further filled through a valve with a saline solution during surgery to achieve the desired breast augmentation.

Both the single shell inflatable mammary prosthesis and the inflatable bilumen mammary prosthesis advantageously permit the size and, in particular, the projection or height of the mammary prosthesis to be varied by altering the amount of fluid admitted to the prosthesis. Such an arrangement is shown in French Patent No. 2199266. However, admitting additional fluid into the prosthesis to increase the mammary prosthesis projection also results in an increase in the mammary prosthesis base diameter, which may be very undesirable. Increasing the mammary prosthesis base dimension once the prosthesis has been inserted may cause tearing of the tissue and possible bleeding. While the projection of the single shell inflatable mammary prosthesis and the inflatable bilumen mammary prosthesis can be varied slightly by the addition of fluid to the prosthesis without severely altering the base dimension of the prosthesis, to obtain a significant increase in the prosthesis projection, it is necessary to employ a prosthesis having a larger molded shell.

In contrast to the prior art mammary prosthesis whose projection may not be significantly increased without a corresponding increase in the prosthesis base dimension, the present invention consists in a mammary prosthesis having a projection which is adjustable, comprising a distensible outer fluid filled container, a distensible inner fluid filled container disposed within the interior of said outer fluid filled container; and a valve in communication with said inner fluid filled container for admitting additional quantities of fluid into said inner fluid filled container to distend said inner fluid filled container and increase its height so as to deform said outer container and to increase the projection of said outer container characterised in that the inner fluid filled container is formed to assume a columnar shape when further inflated by the introduction of additional fluid to increase the projection of said outer container without altering the prosthesis base dimension.

In one preferred embodiment of the invention, an improved, adjustable basis mammary prosthesis comprises a physiologically inert outer container which is formed of a tear-drop shaped shell whose opening is sealed by a disc. Prior to filling the outer container with a physiologically inert fluid, such as silicon gel, a pillow shaped, physiologically inert, fluid filled container is disposed within the outer container beneath the top of the interior surface and is supported from the disc by an extensible, partially fluid filled, column shaped container adhered at its bottom to the disc. A valve is disposed through the disc in communication with the column shaped container to allow an inert fluid, such as a silicone gel, to be admitted into the column shaped container. The addition of more fluid into the column shaped container causes the column shaped container to urge the pillow shaped container against the outer shell so as to deform the outer shell and increase the mammary prosthesis projection.

Alternatively, the mammary prosthesis of the present invention can be constructed of a hollow tear-drop shaped container whose opening is sealed by a disc as before. A single, partially fluid filled, column shaped extensible container is disposed within the interior of the outer shell so as to be beneath, but not in contact with the top interior surface' of the outer container. A valve communicates through the outer container with the inner container so that additional fluid can be admitted to the inner container. When additional fluid is admitted into the inner column shaped container, the column shaped container expands axially to vertically deform the outer container, thereby increasing the prosthesis projection without altering the prosthesis base dimension.

It is an object of the present invention to provide an improved mammary prosthesis having an adjustable projection.

It is yet another object of the present invention to provide an improved mammary prosthesis whose projection can be significantly increased without altering the prosthesis base dimension.

The features of the invention believed to be novel are set forth with particularity in the appended claims. However, the invention itself, both as to organization and method of operation, together with further objects and advantages thereof may best be understood by reference to the following description taken in conjunction with the accompanying drawings in which:

Fig. 1 is a perspective view of a preferred embodiment of a mammary prosthesis having an adjustable basis;

Fig. 2 is a cross-sectional view of the mammary prosthesis of Fig. 1 in its semi-inflated state;

Fig. 3 is a cross-sectional view of the mammary prosthesis of Fig. 1 in its inflated state;

Fig. 4 is a cross-sectional view of an alternate preferred embodiment of a mammary prosthesis shown in its semi-inflated state; and

Fig. 5 illustrates the mammary prosthesis of Fig. 4 in its inflated state.

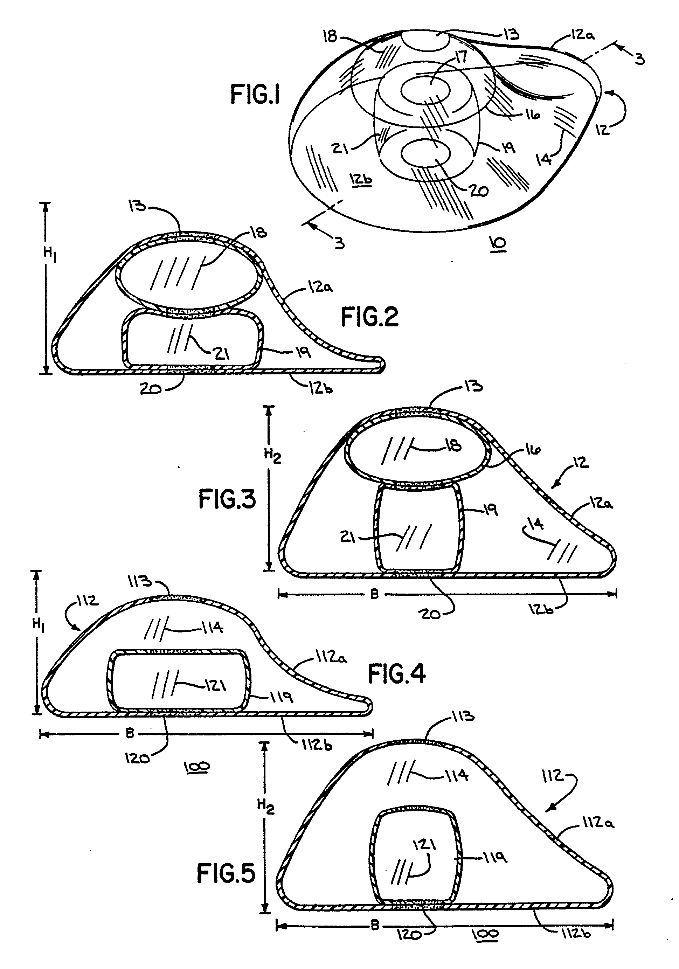

Referring now to the Figures, an improved mammary prosthesis 10 is illustrated in perspective view in Figure 1 and is shown in cross sectional elevation in its semi-inflated and inflated states in Figs. 2 and 3, respectively. Prosthesis 10 is comprised of a distensible container 12 configured of a shell or envelope 12a whose opening is sealed by a disc 12b. Both shell 12a and disc 12b are manufactured from a physiologically inert (safe) material such as silicone rubber or the like. Shell 12a, which typically has relatively thick interior walls, may have either a teardrop shape as illustrated or a round or hemispherical shape depending on the desired mammary augmentation. The interior void of shell 12a is filled through a seal 13 located at the uppermost part of the shell 12a with physiologically inert non-compressible fluid 14, such as dextran, saline, or a silicone gel. Generally, silicone gel is preferred, because when shell 12a is filled with silicone gel 14, the resulting prosthesis allows a mammary augmentation which is more natural in appearance and behavior than would be achieved with a saline or dextran filled shell.

Disposed within the void of shell 12a prior to filling with gel 14 is an interior container 16 which, like shell 12a and disc 12b, is constructed of an inert, physiologically safe, elastomeric material such as silicone rubber. Unlike shell 12a, which has relatively thick walls, container 16, which is typically pillow-shaped, has relatively thin walls allowing container 16 to be extremely pliable which, as will be explained hereinafter, enables the projection of the prosthesis 10 to be adjusted. Container 16 is provided with a valve 17 at its bottom through which a physiologically inert fluid 18, such as silicone gel, is admitted into the container.

Container 16 is supported from disc 12b within the void of shell 12a beneath seal 13 so as to be beneath the "tear drop" of shell 12a by another interior container 19, which is typically column shaped. Container 19, like container 16, is configured of an inert, physiologically safe, elastomeric material such as silicone rubber. Like shell 12a, the walls of container 19 are relatively thick. A valve 20, typically a cylinder valve, is disposed through disc 12b in communication with the bottom of shell 19 to enable fluid such as silicone gel 21 to be admitted into the interior hollow of the container.

In practice, container 19 is initially filled with a small volume of silicone gel 21 through valve 20 so that the total height of containers 16 and 19 equals the projection $H_1$, that is to say, the height of shell 12a as illustrated in Fig. 2. During surgery, the projection of prosthesis 10 can be increased to $H_2$ by expanding container 19 along its axis by the admission of a saline solution 22 into container 19 through valve 20 so as to urge container 16 upwardly against the interior wall of shell 12a, as illustrated in Fig. 3. As container 16 is urged against the interior of shell 12a by container 19, the shell 12a is deformed so as to increase the shell projection. As can now be appreciated, the pliability of container 16, as a consequence of its relatively thin walls, permits the outer shell 12a to be more evenly deformed which is very desirable.

In contrast to a prior art inflatable mammary prosthesis whose base dimension is likely to be altered when the prosthesis projection is altered by increasing the outer shell volume through the addition of more fluid into the outer shell, the projection, of the mammary prosthesis 10 of the present invention can be increased without any increase in the base dimension B of the prosthesis as illustrated in Fig. 3. When the column shaped container 19 is filled with a volume of saline fluid 22 to urge the pillow-shaped container 16 against the interior surface of shell 12a to deform the shell, the overall volume of shell 12a is not substantially increased. Rather, only the height or projection of the shell 12a changes. Since the shell volume does not substantially change, the shell base dimension B does not change significantly.

The ability of prosthesis 10 to exhibit a substantially constant base dimension notwithstanding adjustments to its projection is very advantageous. Generally, the base dimension for an implant is selected in accordance with the diameter of the breast to be augmented. If the base dimension is unknowingly altered by virtue of prosthesis inflation, as will likely occur with prior art mammary prostheses when attempts are made to increase the prosthesis projection by the addition of fluid into the outer shell, then it is likely that resultant augmentation may not appear natural.

Mammary prosthesis 10 is readily constructed in the following manner. First, the column-shaped container 19 is partially filled with gel 21 through valve 20 and the valve is then closed. The pillow-shaped container 16 is then adhered to the top of container 19 so that seal 17 is in contact with container 19 and container 16 is thereafter filled with silicone gel. Next, both containers 16 and 19 are debubbled and then baked to cure or vulcanize the silicone gel within each container.

Following the step of baking, the sub-assembly of the column container 19 and pillow-shaped container 16 are then disposed within the inside of the outer shell 12a so as to be beneath seal 13 and then the column shaped container 19 is adhered to disc 12b. After containers 16 and 19 are disposed within shell 12a and container 19 is adhered to disc 12b the disc 12b is secured to shell 12a. The resultant structure is then baked. Thereafter, shell 12a and container 19 are filled with silicone gel, and then the entire prosthesis is debubbled and then baked. Finally, the valve 20 is itself filled with silicone gel and is sealed so that the prosthesis is now ready for subsequent use.

An alternative preferred embodiment 100 of a mammary prosthesis having an adjustable basis is illustrated in cross sectional elevation in its semi-inflated and its inflated states in Figs. 4 and 5, respectively. Prosthesis 100, like prosthesis 10, is comprised of a hollow extensible container 112, typically teardrop in shape, the container being configured of a shell 112a made from an elastomeric inert material such as silicone rubber, and an elastomeric, 112b which seals the opening into shell 112a. At the top of shell 112a that is, directly beneath the "tear drop" of shell 112a, is a seal 113 which allows an inert fluid 114, typically a silicone gel to be admitted into the void of shell 112a. Prior to adhering disc 112a to shell 112a and filling the shell with an inert fluid 114, an inner axially expandable container 119, typically comprised of a column-shaped elastomeric shell 119, is adhered to disc 112b so as to be directly beneath seal 113 of shell 112a. A valve 120 is disposed through disc 112B so as to be in communication with column shaped container 119 for admitting an inert fluid 121, such as silicone gel, into container 119 so that the container is partially filled.

The projection or height $H_1$ of prosthesis 100 in its semi-inflated state, illustrated in Fig. 4, may be readily increased to $H_2$ as illustrated in Fig. 4 during augmentation mammoplasty by admitting saline fluid or the like into container 119 through valve 120 so that the container is deformed along its axis so as to deform envelope 112a vertically, as illustrated in Fig. 5, thereby increasing the prosthesis projection. The base dimension B of prosthesis 100 remains substantially the same notwithstanding the addition of further fluid into container 119 because, as the container 119 is deformed along its vertical axis, shell 112a is deformed vertically as opposed to horizontally. In comparison with prosthesis 10 described with respect to Figs. 1 and 2, prosthesis 100 is all but identical except that prosthesis 100 obviates the need for an interior pillow shaped container such as pillow shaped container 16 of Figs. 1, 2 and 3. Thus, prosthesis 100 of Figs. 4 and 5 is less expensive to fabricate and manufacture than prosthesis 10 of Figs. 1, 2 and 3.

Prosthesis 100 is constructed in a manner very much similar to the construction of prosthesis 10 described earlier. However, owing to the lack of any pillow shaped container within the prosthesis, the steps of adhering the pillow shaped container to the column shaped container and filling the pillow shaped container with silicone gel are thus not required.

The foregoing describes an improved mammary prosthesis whose projection can be readily adjusted by inflating an inner shell within the prosthesis without altering the prosthesis base dimension.

## Claims

1. A mammary prosthesis (10, 100) having a projection which is adjustable, comprising a distensible outer fluid filled container (12, 112), a distensible inner fluid filled container (19, 119) disposed within the interior of said outer fluid filled container (12, 112); and a valve (20/120) in communication with said inner fluid filled container (19, 119) for admitting additional quantities of fluid into said inner fluid filled container (19, 119) to distend said inner fluid filled container (19, 119) and increase its height so as to deform said outer container (12, 112) and to increase the projection of said outer container (12, 112) characterised in that the inner fluid filled container (19, 119) is formed to assume a columnar shape when further inflated by the introduction of additional fluid to increase the projection ($H_2$) of said outer container (12, 112) without altering the prosthesis base dimension (B).

2. The prosthesis (10) of claim 1 wherein a second distensible inner fluid filled container (16) is disposed between the top of the first inner fluid filled container (19) and the inside of the top of the outer fluid filled container (12).

3. The prosthesis (10, 100) of claim 1, wherein the outer distensible fluid filled container (12, 112) has a shell (12a) of physiologically inert elastomeric material.

4. The prosthesis of claim 1 wherein the outer container (12, 112) is tear-shaped.

5. The prosthesis of claim 1 wherein the outer container (12, 112) is filled with a silicone gel (21).

6. The prosthesis of claim 1 wherein the distensible, inner fluid filled container (19, 119) is partially filled with silicone gel (21).

7. The prosthesis of claim 2 in which the second distensible inner fluid filled container (16) is pillow-shaped.

8. The prosthesis of claim 2 in which the second distensible inner fluid filled container (16) includes a valve (17) for admitting additional fluid.

## Patentansprüche

1. Brustprothese (10, 100) mit einem vorspringenden Element, das einstellbar ist und einen dehnbaren äusseren, mit einem Strömungsmittel gefüllten Behälter (12, 112) und einen dehnbaren inneren, mit einem Strömungsmittel gefüllten Behälter (19, 119) aufweist, der im Inneren des äußeren, mit dem Strömungsmittel gefüllten Behälters (12, 112) angeordnet ist, und mit einem Ventil (20, 120), das mit dem inneren, mit dem Strömungsmittel gefüllten Behälter (19, 119) in Verbindung steht, um in den inneren, mit dem Strömungsmittel gefüllten Behälter (19, 119) zusätzliche Mengen an Strömungsmittel einzulassen, damit dieser Behälter gedehnt und seine Höhe vergrößert wird, so daß der äußere Behälter (12, 112) verformt wird und das vorspringende Element des äußeren Behälters (12, 112) vergrößert wird, dadurch gekennzeichnet, daß der innere, mit Strömungsmittel gefüllte Behälter (19, 119) so geformt ist, daß er eine säulenförmige Gestalt annimmt, wenn er durch die Einführung von zusätzlichem Strömungsmittel zur Vergrößerung des vorspringenden Elementes ($H_2$) des äußeren Behälter (12, 112) weiter aufgeblasen wird, ohne daß hierdurch die Basisabmessung (B) der Prothese verändert wird.

2. Brustprothese nach Anspruch 1, bei der ein zweiter dehnbarer innerer, mit einem Strömungsmittel gefüllter Behälter (16) zwischen dem oberen Ende des ersten inneren, mit Strömungsmittel gefüllten Behälters (19) und der Innenseite des oberen Endes des äußeren, mit Strömungsmittel gefüllten Behälters (12) angeordnet ist.

3. Brustprothese nach Anspruch 1, bei der der äußere dehnbare, mit Strömungsmittel gefüllte Behälter (12, 112) eine Hülle (12a) aus einem physiologisch inerten elastomeren Material besitzt.

4. Brustprothese nach Anspruch 1, bei der der äußere Behälter (12, 112) tropfenförmig ausgebildet ist.

5. Brustprothese nach Anspruch 1, bei der der äußere Behälter (12, 112) mit einem Silikongel (21) gefüllt ist.

6. Brustprothese nach Anspruch 1, bei der der dehnbare innere, mit Strömungsmittel gefüllte Behälter (19, 119) teilweise mit Silikongel (21) gefüllt ist.

7. Brustprothese nach Anspruch 2, bei der der zweite dehnbare innere, mit Strömungsmittel gefüllte Behälter (16) kissenförmig ausgebildet ist.

8. Brustprothese nach Anspruch 2, bei der zweite dehnbare innere, mit Strömungsmittel gefüllte Behälter (16) ein Ventil (17) zum Einlassen von zusätzlichem Strömungsmittel aufweist.

## Revendications

1. Prothèse mammaire (10, 100) ayant une projection qui est règlable, comprenant un réservoir externe dilatable (12, 112) rempli de fluide, un réservoir interne dilatable (19, 119) remplie de fluide placé à l'intérieur dudit réservoir externe (12, 112) rempli de fluide; et une soupape (20/120) en communication avec ledit réservoir interne (19, 119) rempli de fluide pour introduire des quantités supplémentaires de fluide dans ledit réservoir interne (19, 119) rempli de fluide pour dilater ledit réservoir interne (19, 119) rempli de fluide, et accroître sa hauteur de manière à déformer ledit réservoir externe (12, 112) et à augmenter la projection dudit réservoir externe (12, 112), caractérisée en ce que le réservoir interne (19, 119) rempli de fluide est formé pour prendre une forme colonnaire lorsqu'il est ultérieurement gonflé par l'introduction de fluide supplémentaire pour augmenter la projection ($H_2$) dudit réservoir externe (12, 112) sans modifier la dimension (B) de la base de la prothèse.

2. Prothèse 10 selon la revendication 1, dans laquelle un second réservoir interne dilatable (16) rempli de fluide, est placé entre le sommet du premier réservoir interne (19) remplie de fluide et l'intérieur de haut du réservoir externe (12) remplie de fluide.

3. Prothèse (10, 100) selon la revendication 1, dans laquelle le réservoir externe dilatable (12, 112) rempli de fluide, comporte une enveloppe (12a) d'un matériau élastomère physiologiquement inerte.

4. Prothèse selon la revendication 1, dans laquelle le réservoir externe (12, 112) est en forme de larme.

5. Prothèse selon la revendication 1, dans laquelle le réservoir externe (12, 112) est rempli d'un gel de silicone (21).

5. Prothèse selon la revendication 1, dans laquelle le réservoir interne, dilatable (19, 119) rempli de fluide est partiellement rempli de gel de silicone (21).

7. Prothèse selon la revendication 2, dans laquelle le second réservoir interne, dilatable (16) rempli de fluide est en forme de coussin.

8. Prothèse selon la revendication 2, dans laquelle le second réservoir interne, dilatable (16) rempli de fluide comprend une soupape (17) pour introduire du fluide supplémentaire.

FIG.1

FIG.2

FIG.3

FIG.4

FIG.5